# EUROPEAN PATENT APPLICATION

(11) **EP 1 275 731 A1**
(43) Date of publication of application: **15.01.2003**
(21) Application number: 01202638.1
(22) Date of filing: 09.07.2001
(51) Int. Cl.: C12P 21/00, G01N 33/92

(54) **Method for preparing lipid II and use of the lipid II thus obtained**

(71) Applicant: Universiteit Utrecht Holding B.V., 3584 CL Utrecht (NL)
(72) Inventor: Breukink, Eefjan, 3584 GS Utrecht (NL)
(74) Representative: Van Someren, Petronella F. H. M.

(57) **Abstract**

The present invention relates to a method for preparing Lipid II, which method comprises the steps of preparing a mixture of bacterial membranes, uridine diphosphate N-acetylmuramyl peptide (UDP-MurNAc), uridine diphosphate N-acetylglucosamine (GlcNAc), a chain of n prenyl residues, wherein n is at least 2, and optionally a detergent in a buffer; reacting the mixture thus obtained to prepare an n-Lipid II containing mixture, wherein n represents the amount of prenyl residues in the Lipid II molecule and wherein n is at least 2; and optionally further purifying the n-Lipid II containing mixture. Preferably n is selected from the range 2-25. Instead of bacterial membranes the reaction mixture may comprise isolated enzymes, such as MraY (undecaprenyl phosphate phospho-N-acetylmuramoyl-pentapeptide transferase).

## Description

The present invention relates to a method for the preparation of Lipid II and to the lipid II thus obtained and its use in the screening for antibacterial compounds.

The spread of infectious diseases and the increase in antibiotic resistance is a life-threatening global development that calls for new approaches to kill microorganisms. Of all the potential targets, the essential and unique pathway of bacterial cell wall synthesis, target of the first known antibiotic penicillin, remains a perfect candidate for development of new antibiotics.

The bacterial cell wall (peptidoglycan layer) consists of polymers of disaccharide units that are cross-linked via peptide bridges. The basic building block of the cell wall, the peptidoglycan subunit, is a disaccharide of amino sugars N-acetylmuramic acid (MurNAc) and N-acetylglucosamine (GlcNAc). The MurNAc sugar contains a pentapeptide needed for the crosslinking.

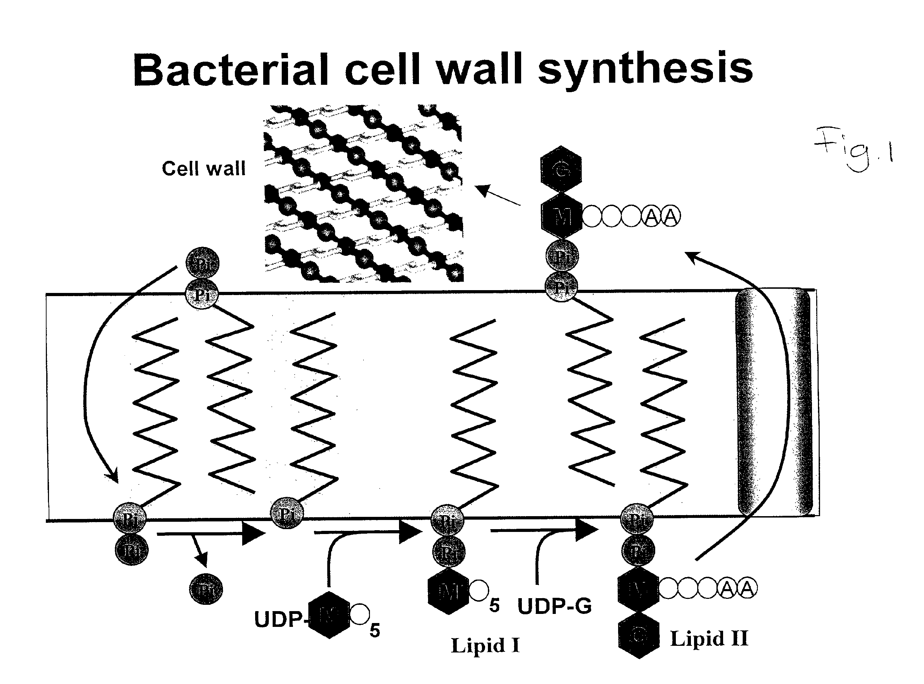

The peptidoglycan subunit is assembled in the cytosol, and it is dependent on a special carrier for its transmembrane transport (Fig. 1). Despite extensive knowledge on the enzymes involved in the synthesis pathway of the cell wall the major question of the mechanism of transmembrane transport of the cell wall subunits remains to be resolved.

The carrier for the subunits is a polyprenol (bactoprenol) and when assembled, the subunit and polyprenol together form the Lipid II molecule. Only the complete Lipid II molecule is transported across the membrane, and thus Lipid II is a key molecule in the bacterial cell wall synthesis. A consequence of such an important role is that it has become a popular target for existing antibiotics and a promising target for new types of antibiotics.

To date there are already several examples of antibiotics acting through interaction with Lipid II. An example is vancomycin, the antibiotic of last resort to treat MRSA infections, which inhibits cell wall synthesis by binding to Lipid II and the same holds for a range of other structurally unrelated antibiotics. Recently the inventors observed a completely different lipid II mediated bacterial killing mechanism for the peptide antibiotic nisin. This peptide kills bacteria by pore formation in the plasma membrane and uses Lipid II as a docking molecule to increase the efficiency by three orders of magnitude.

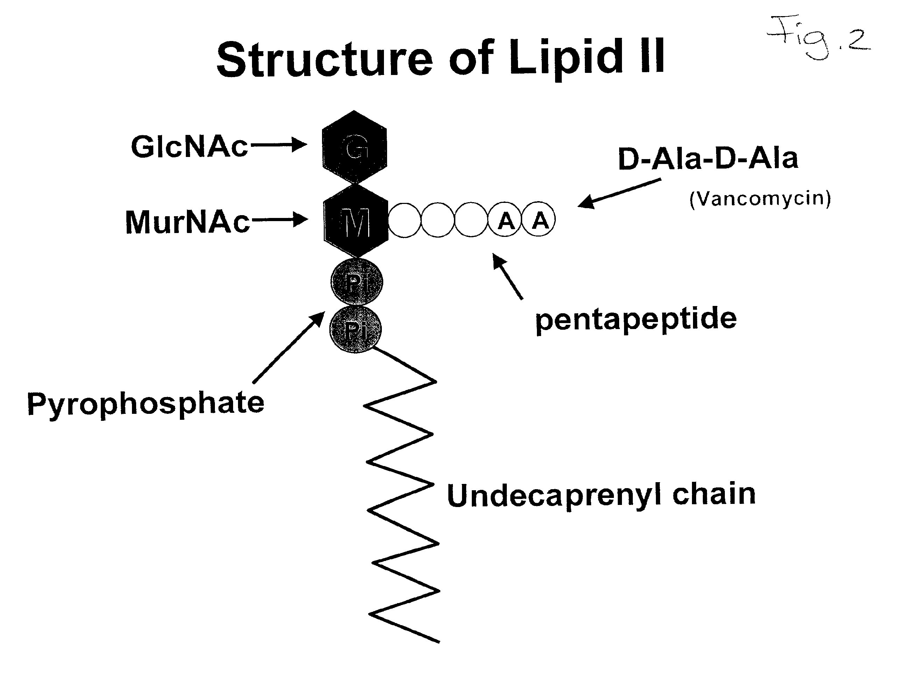

From a chemical point of view Lipid II is a unique molecule: it combines carbohydrate, peptide and lipid all in one molecule (Fig. 2). The two sugars show little or no variation throughout the eubacteria, which is an advantage since it thus provides a common target for a possible broadspectrum antibiotic. There is variation in the pentapeptide (mainly in the third residue) which consists of alternating L- and D-amino acids. The pyrophosphate that links the subunit to the polyprenol is (after transport across the membrane) probably the only pyrophosphate that is exposed to the exocytoplasmic side of the plasma membrane of bacteria.

The carriers for membrane transport of complex sugars are usually long polyprenols, like the undecaprenyl chain of Lipid II in the case of the bacterial cell wall synthesis. Those polyprenols are long enough to easily span a membrane in extended conformation.

Thus far attempts to unravel the mechanism of membrane transport of the peptidoglycan subunit have failed. To a large extent this failure can be attributed to the low content of endogenous bactoprenol in bacterial membranes. On top of this, Lipid II is a metabolite with a short half-life, complicating isolation of sufficient amounts. Information on the mode of action of antibiotics that act via interaction with Lipid II is limited and often incomplete for the same reason.

The availability of large amounts of Lipid II will be invaluable for studying the interaction of antibiotics with Lipid II up to the atomic level. Likewise, the chances to unravel the mechanism of membrane transport would be increased dramatically.

There is thus a need for large amounts of lipid II to study the transport mechanism, to elucidate the structures of antibiotics in complex with Lipid II, to design new antibiotics and to develop new technologies usable for screening for new antibiotics.

The research that led to the invention has now provided a straightforward and flexible semi-synthesis of Lipid II that allows for the purification of up to and even beyond 100 mg quantities.

The method of the invention for preparing Lipid II, comprises the steps of:
a) preparing a mixture of bacterial membranes, uridine diphosphate N-acetylmuramyl peptide (UDP-MurNAc), uridine diphosphate N-acetylglucosamine (GlcNAc), a chain of n prenyl residues, wherein n is at least 2, and optionally a detergent in a buffer;
b) reacting the mixture thus obtained to prepare an n-Lipid II containing mixture, wherein n represents the amount of prenyl residues in the Lipid II molecule and wherein n is at least 2; and
c) optionally further purifying the n-Lipid II containing mixture.

Preferably n is choosen from the range 2-25. However, larger chains of prenyl residues, e.g. as they occur in nature, can also be used.

In an alternative embodiment of the invention, the use of bacterial membranes for the synthesis may be omitted when the enzymes MurG (UDP-N-acetylglucosamine-N-acetylmuramyl-(pentapeptide) pyrophosphorylundecaprenol N-acetylglucosamine transferase) and MraY (undecaprenyl phosphate phospho-N-acetylmuramoyl-pentapeptide transferase) are used instead.

The n-Lipid II containing mixture can be used as such for screening applications as will be described hereinbelow. However, it is also possible to further purify the thus obtained n-Lipid II containing mixture. In case n is 2 or 3 the mixture can be further purified by precipitating the bacterial membranes from the mixture. 2-Lipid II and 3-Lipid II are soluble molecules that remain in solution upon precipitation of the membranes used for their production.

In case n is 4-25 the mixture can be further purified by butanol/pyridine extraction and optional ion exchange chromatography yielding purified n-Lipid II. n-Lipid II molecules in which n is 4-25 are no longer soluble.

The use of the detergent in the preparation process is dependent on the number of prenyl residues in the prenyl chain. When n is 2 or 3 a detergent is not necessary. The detergent is however essential in case n is 4-25. It was found that the conversion of the prenyl chain is dependant on the detergent concentration.

The detergent is preferably octylglucoside in a concentration in the reaction mixture of at least 0.4%, preferably at least 0.6%, more preferably at least 0.8%. Octylglucoside is a mild and uncharged detergent. The detergent may be added to the reaction mixture before or at the same time as the prenyl chain. When n is 4-25, the prenyl chain is preferably dissolved in the detergent after which this is added to a mixture of the other reaction components.

An alternative detergent is Triton X-100, which can be used in a concentration in the reaction mixture which is at least 0.2%, preferably at least 0.3%, more preferably at least 0.4% (w/v).

The bacterial membranes are preferably derived from Micrococcus flavus, which comprises naturally a tenfold of Lipid II molecules as compared to E.coli. M.flavus does also comprise the enzymes that are involved in the synthesis process of n-Lipid II. When the bacterial membranes are derived from other bacteria than Micrococcus flavus extra enzymes, i.e. MurG (or UDP-N-acetylglucosamine-N-acetylmuramyl-(pentapeptide) pyrophosphorylundecaprenol N-acetylglucosamine transferase), are to be added to the reaction mixture. The bacterial membranes that are not derived from M. flavus are preferably from Gram-positive bacteria, such as Bacillus subtilis or Lactococcus lactis.

It was demonstrated according to the invention that Lipid II with various lengths of the prenyl chain can be produced. Of these n-Lipid II, wherein n is 2-10 or 12-25 or even longer, are novel. These molecules are obtainable by means of a method of the invention.

In essence, any molecule that either binds Lipid II with high affinity, or resembles Lipid II such that it blocks or competes for the binding sites on the enzymes involved in the cell wall synthesis is a very good candidate for an antibiotic. The simplest and most reliable approach to discover novel antibiotics is to develop screening methods based on the mechanism of action of a known drug. This in combination with a fast selection method for Lipid II binding can be used to deliver an effective and totally new class of antibiotics. Furthermore, the flexible semi-synthesis of Lipid II according to the invention makes it possible to obtain specifically labeled (water-soluble) Lipid II molecules. It can also be used to convert a polyprenylphosphate attached to a solid support to Lipid II. Both the water-soluble and the immobilized Lipid II can be used to test the synthesized peptides for Lipid II binding in a rapid and efficient way to identify the antibiotic candidates. Moreover, this approach can be used to fish for Lipid II binding proteins.

All these n-Lipid II molecules, wherein n is 2-25 or longer, can thus be used in the screening of potential antibacterial compounds. Various methods of screening can be designed. A method that is based on Lipid II integrated into micelles, for example comprises the steps of:
a) providing labeled n-Lipid II, wherein n is 4-25;
b) combining the labeled n-Lipid II with a detergent to prepare micelles;
c) contacting the thus obtained micelles with a range of compounds to be tested coupled to a solid substrate; and
d) identifying the location of the label on the solid substrate to determine which test compound(s) has or have bound to the micelles.

The label is preferably provided in or onto the peptide chain in the n-Lipid II molecule. Methods for labeling comprise for example those described in Weppner, W.A. and Neuhaus, F.C. (1977) J. Biol. Chem. 252, 2296-2303, which describes the labeling of UDP-MurNAc-pentapeptide with Dansylchloride. Any label with an amino, or carboxyl reactive group can however be used for the labeling of n-Lipid II. Preferably, the UDP-MurNac-pentapeptide is labeled first before the Lipid II synthesis begins. As an alternative, Lipid II can be directly labeled.

In the micelles, the sugar and peptide part of the n-Lipid II molecules are exposed. This method can be used to find compounds that bind to these exposed parts.

Alternatively, a method for screening of potential antibacterial compounds comprises the steps of:
a) providing labeled n-Lipid II, wherein n is 2 or 3;
b) contacting the thus labeled n-Lipid II with a range of compounds to be tested coupled to a solid substrate; and
c) identifying the location of the label on the solid substrate to determine which test compound(s) has or have bound the labeled n-Lipid II.

In this situation the Lipid II molecules are soluble.

A further embodiment of a method for screening of potential antibacterial compounds comprises the steps of:
a) providing n-Lipid II, wherein n is 2-25, bound to a solid substrate;
b) preparing a column of the n-Lipid II bound to the solid substrate;
c) contacting a mixture of compounds to be tested by eluting it through the column thereby inducing binding between compounds in the mixture and n-Lipid II;
d) washing the column to remove unbound material and eluting and identifying the compounds that were bound to the n-Lipid II.

The antibacterial compounds identified by means of one or more of the above methods are also part of this invention.

When used in this application the term "n-Lipid II" is intended to mean Lipid-II type molecules in which the prenyl side chain may vary in the amount of isoprene units. The minimum amount of isoprene units is 2, whereas preferably the maximum is 25. However, longer prenyl chains are also part of this invention. Fig. 2 shows a 11-Lipid II molecule having an undecaprenyl chain.

The peptide part may vary in length and composition of the amino acids. Preferably the peptide part is a pentapeptide consisting of alternating L- and D-amino acids, for example having the sequence L-Ala-D-Glu-γ-L-Lys-D-Ala-D-Ala. The lysine residue at position 3 of the pentapeptide provides a great tool for the attachment of a functional group. Depending on the functional group chosen this will for example allow for labeling with a fluorescent label.

The sugars are in Eubacteria always D-GlcNAc and D-MurNAc.

The present invention will be further illustrated in the following examples, which are not intended to limit the invention. In the examples and the rest of this application reference is made to the following figures:
**Figure 1**: Bacterial cell wall synthesis
**Figure 2:** Structure of Lipid II
**Figure 3:** Elution profile of anion-exchange chromatography of lipids prepared according to the invention using DEAE-cellulose (acetate form) and a linear gradient from 0-0.4 M ammonium acetate, visualized by spotting samples from the fractions on TLC, and developing in CHCl₃/methanol/water/amonia (88:48:10:1). The lipids were visualized by iodine staining.
**Figure 4**: Determination of the amount of membranes necessary for the complete conversion of the undecaprenylphosphate to 11-Lipid II.
**Figure 5:** Determination of the octylglucoside concentration necessary for the complete conversion of the undecaprenylphosphate to 11-Lipid II.
**Figure 6:** Test of various detergents in the Lipid II synthesis of the invention. Lane 1 = lubrol, lane 2 = Brij 35, lane 3 = Tween 20, lane 4 = Triton X-100, lane 5 = dodecylmaltoside, lane 6 = N,N dimethyldodecylamine-N-oxide, lane 7 = CHAPS, lane 8 = SDS and lane 9 = octylglucoside.
**Figure 7:** Comparison between the detergents octylglucoside (lanes 1-5) and Triton X-100 (lanes 6-10) with increasing membrane concentrations. Lanes 1 and 6: 4 nmol lipid Pi; lanes 2 and 7: 8 nmol lipid Pi; lanes 3 and 8: 20 nmol lipid Pi; lanes 4 and 9: 40 nmol lipid Pi; and lanes 5 and 10: 80 nmol lipid Pi.
**Figure 8:** Determination of the Triton X-100 concentration necessary for the complete conversion of the undecaprenylphosphate to 11-Lipid II. Lane 1: 0.2 % (w/v); lane 2: 0.4% (w/v); lane 3: 0.6% (w/v); lane 4: 0.8% (w/v); and lane 5: 1.0% (w/v).

### EXAMPLES

### EXAMPLE 1

### Synthesis of 11-Lipid II

A reaction mixture was prepared of M. flavus membranes [isolated according to Konings, W.N., Bisschop, A., Veenhuis, M., Vermeulen, C.A. J. (1973) Bacteriol 116, 1456-1465] containing in total 120 nmol phospholipids, 100 nmol UDP-MurNAc-pentapeptide, 100 nmol UDP-GlcNAc, 100 mM Tris-HCl pH 8.0, 0.3 mM MgCl₂, 0.8 % octylglucoside, and 15 nmol undecaprenyl-phosphate (added from a stock solution of 1 mM in 2% octylglucoside, 100 mM Tris-HCl, pH 8.0). The reaction mixture was kept at room temperature for 2 hours.

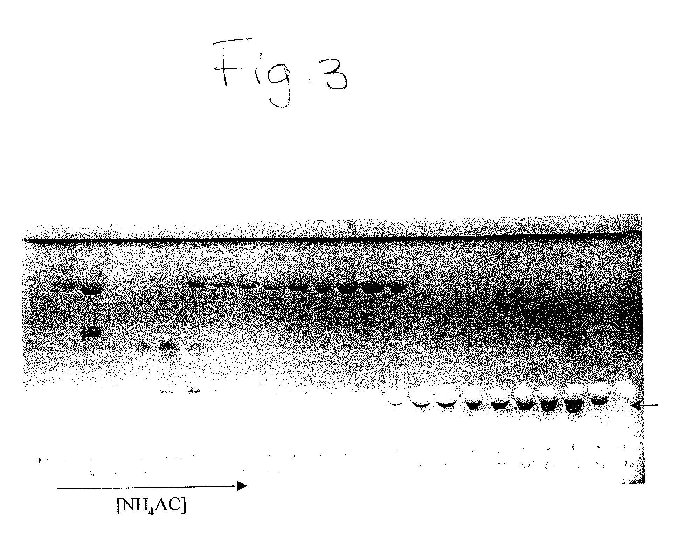

Subsequently the reaction mixture was extracted with butanol/pyridine acetate, pH 4.2, after which the butanol phase was washed with water. The butanol phase was evaporated and the remaining residue dissolved in CHCl₃/methanol/water (2:3:1) and purified by anion-exchange chromatography using DEAE-cellulose (acetate form) and a linear gradient from 0-0.4 M ammonium acetate. Fig. 3 shows the resulting elution profile, visualized by spotting samples from the fractions on TLC, and developed in CHCl₃/methanol/water/amonia (88:48:10:1). The lipids were visualized by iodine staining.

### EXAMPLE 2

### Variations in process parameters

### 1. Amount of vesicles

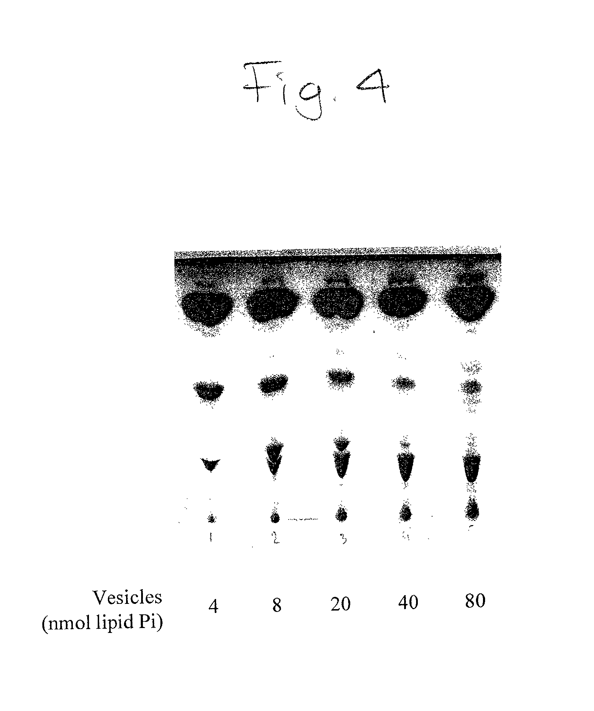

The amount of vesicles was varied to determine the minimum amount of vesicles necessary to produce n-Lipid II. The amounts tested were 4, 8, 20, 40 and 80 nmol lipid Pi. It was found that an amount of membranes corresponding to at least 80 nmol lipid-phosphate was necessary for complete conversion of the undecaprenylphosphate to 11-Lipid II (Fig. 4).

### 2. Substrates for n-Lipid II synthesis

It was tested whether polyprenyls of more than 11 isoprene units and smaller molecules of less units could also be converted to n-Lipid II by means of the method of the invention. The substrates tested were:
- 1-P: Isopentenyl Monophosphate
- 1'-P: γ,γ-Dimethylallyl Monophosphate
- 2-P: 3,7-Dimethyl-2,6-octadien-1-yl Monophosphate (Geranylphosphate)
- 3-P: 3,7,11-Trimethyl-2,6,10-dodecatrien-1-yl Monophosphate (Farnesylphosphate)
- 4-P: 3,7,11,15-Tetramethyl-2,6,10,14-hexadecatetraen-1-yl Monophosphate
- 4'-P: 3,7,11,15-Tetramethyl-2-hexadecen-1-yl Monophosphate (Phytylphosphate)
- 7-P: Heptaprenyl Monophosphate (C30-Prenyl Monophosphate)
- 8-P: Octaprenyl Monophosphate (C40-Prenyl Monophosphate)
- 11-P: Undecaprenyl Monophosphate (C55-Prenyl Monophosphate)
- 15-P: Pentadecaprenyl Monophosphate (C75-Prenyl Monophosphate)
- 18-P: Octadecaprenyl Monophosphate (C90-Prenyl Monophosphate)
- 18'-P: α-Dihydrooctadecaprenyl Monophosphate (C90-Dihydroprenyl Monophsophat)
- 21-P: Heneicosaprenyl Monophosphate (C105-Prenyl Monophosphate)
- 25-P: Pentacosaprenyl Monophosphate (C125-Prenyl Monophosphate)

It was found that 1-P was not a substrate in the reaction. However molecules up to 25-P all yielded n-Lipid II production. Of these 2-Lipid II and 3-Lipid II could not be purified with butanol/pyridine because they are water soluble and do not end up in the butanol phase.

### 3. Amount of detergent

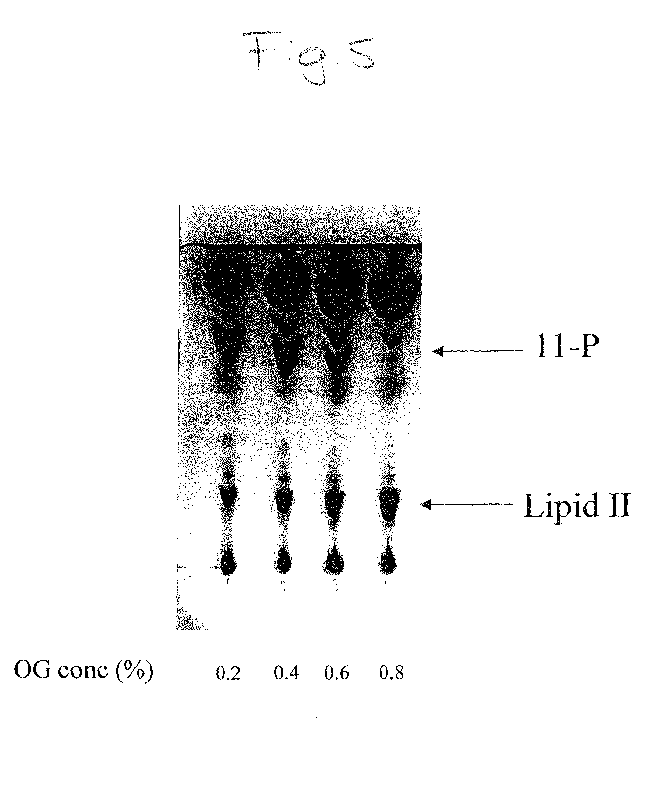

To test the effect of the amount of detergent various concentrations of octylglucoside were used in the basic method described in Example 1. These concentrations are 0.2, 0.4, 0.6 and 0.8%. It was found that 0.8% octylglucoside results in a 100% conversion of 11-P into 11-Lipid II (Fig. 5)

### 4. Type of detergent

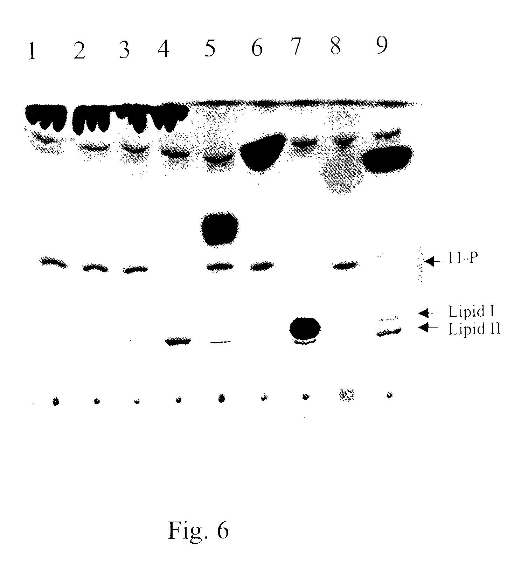

To test whether other detergents than octylglucoside are also suitable in the method of the invention, a range of detergents (1%) was used in the basic method described in Example 1. Figure 6 shows the results. It follows from this figure that besides octylglucoside, Triton X-100 and CHAPS are useful. Because CHAPS overlaps with Lipid II on the gel, Triton X-100 was selected for further study.

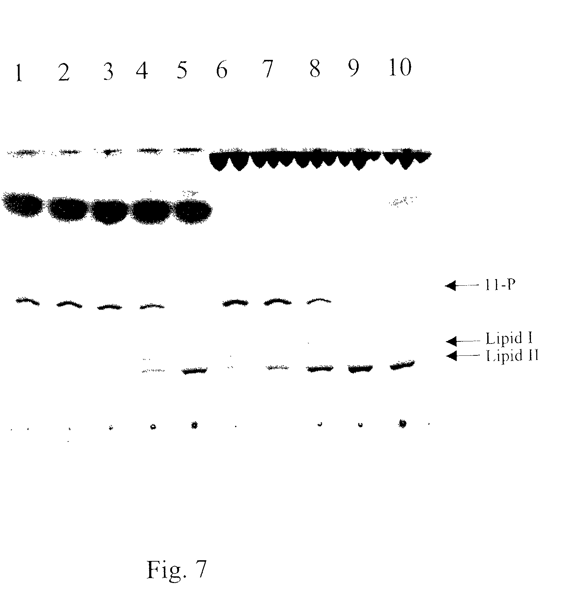

In the basic method of Example 1, octylglucoside and Triton X-100 were used in a 1% concentration with increasing membrane concentrations. Figure 7 shows the results. It follows that Triton X-100 is suitable as a detergent and needs less membranes (i.e. enzymes) for a complete conversion than the reaction with octylglucoside.

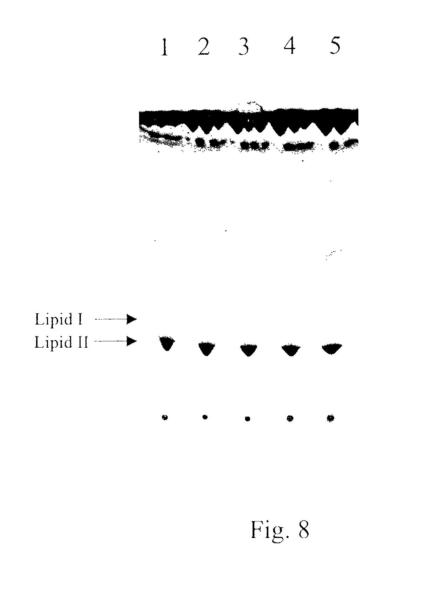

The concentration dependency of Triton X-100 was determined as described above for octylglucoside. It follows from Fig. 8 that at a concentration of 0.4% (w/v) a complete conversion of 11-P to 11-Lipid II is observed.

## Claims

1. Method for preparing Lipid II, which method comprises the steps of:
a) preparing a mixture of bacterial membranes, uridine diphosphate N-acetylmuramyl peptide (UDP-MurNAc), uridine diphosphate N-acetylglucosamine (GlcNAc), a chain of n prenyl residues, wherein n is at least 2, and optionally a detergent in a buffer;
b) reacting the mixture thus obtained to prepare an n-Lipid II containing mixture, wherein n represents the amount of prenyl residues in the Lipid II molecule and wherein n is at least 2; and
c) optionally further purifying the n-Lipid II containing mixture.

2. Method as claimed in claim 1, wherein n is selected from the range 2-25.

3. Method as claimed in claim or 2, wherein no bacterial membranes are present in the mixture, but the mixture does contain the enzyme MraY (undecaprenyl phosphate phospho-N-acetylmuramoyl-pentapeptide transferase) instead.

4. Method as claimed in claims 1-3, wherein in case n is 2 or 3 the mixture is further purified by precipitating the bacterial membranes from the mixture and/or optionally by HPLC.

5. Method as claimed in claim 1-3, wherein in case n is 4-25 the mixture is further purified by butanol/pyridine extraction and optional ion exchange chromatography yielding purified n-Lipid II.

6. Method as claimed in claims 1-3 and 5, wherein in case n is 4-25, the detergent is present.

7. Method as claimed in claim 6, wherein the detergent is octylglucoside.

8. Method as claimed in claim 7, wherein the octylglucoside concentration in the reaction mixture is at least 0.4%, preferably at least 0.6%, more preferably at least 0.8%.

9. Method as claimed in claim 6, wherein the detergent is Triton X-100.

10. Method as claimed in claim 9, wherein the Triton X-100 concentration in the reaction mixture is at least 0.2%, preferably at least 0.3%, more preferably at least 0.4%.

11. Method as claimed in claims 1-10, wherein the bacterial membranes are derived from Micrococcus flavus.

12. Method as claimed in claims 1-10, wherein the bacterial membranes are derived from other bacteria than Micrococcus flavus and the mixture then comprises extra enzymes, in particular MurG (UDP-N-acetylglucosamine-N-acetylmuramyl-(pentapeptide) pyrophosphoryl-undecaprenol N-acetylglucosamine transferase).

13. Method as claimed in claim 12, wherein the other bacteria are Gram-positive bacteria.

14. Method as claimed in claims 12 and 13, wherein the bacteria are Bacillus subtilis or Lactococcus lactis.

15. n-Lipid II, wherein n is 2-10 or 12-25, obtainable by means of a method as claimed in claims 1-14.

16. n-Lipid II, wherein n is 2-25, for use in the screening of potential antibacterial compounds.

17. Method for screening of potential antibacterial compounds comprising the steps of:
a) providing labeled n-Lipid II, wherein n is 4-25;
b) combining the labeled n-Lipid II with a detergent to prepare micelles;
c) contacting the thus obtained micelles with a range of compounds to be tested coupled to a solid substrate; and
d) identifying the location of the label on the solid substrate to determine which test compound(s) has or have bound to the micelles.

18. Method for screening of potential antibacterial compounds comprising the steps of:
a) providing labeled n-Lipid II, wherein n is 2 or 3;
b) contacting the thus labeled n-Lipid II with a range of compounds to be tested coupled to a solid substrate; and
c) identifying the location of the label on the solid substrate to determine which test compound(s) has or have bound the labeled n-Lipid II.

19. Method for screening of potential antibacterial compounds comprising the steps of:
a) providing n-Lipid II, bound to a solid substrate;
b) contacting the thus bound n-Lipid II with a mixture of compounds to be tested to inducing binding between compounds in the mixture and n-Lipid II;
c) preparing a column of the solid substrate having bound thereon compounds in the mixture;
d) eluting and identifying the compounds that were bound to the n-Lipid II.

20. Antibacterial compound identified by means of one or more of the methods as claimed in claims 17-19.
